# EUROPEAN PATENT SPECIFICATION

(11) **EP 1 937 826 B1**
(45) Date of publication and mention of the grant of the patent: **15.04.2009**
(21) Application number: 06806395.7
(22) Date of filing: 19.10.2006
(51) Int. Cl.: C12P 21/00, C12P 7/62

(54) **ENZYMATIC CONVERSION OF OLIGOPEPTIDE AMIDES TO OLIGOPEPTIDE ALKYL ESTERS**
ENZYMATISCHE UMSETZUNG VON OLIGOPEPTIDAMIDEN ZU OLIGOPEPTIDALKYLESTERN
CONVERSION ENZYMATIQUE D'AMIDES D'OLIGOPEPTIDES EN ALKYL ESTERS D'OLIGOPEPTIDES

(30) Priority: 20.10.2005 EP 05077419
(43) Date of publication of application: 02.07.2008
(73) Proprietor: DSM IP Assets B.V., 6411 TE Heerlen (NL)
(72) Inventor: QUAEDFLIEG, Peter, Jan, Leonard, Mario, NL-6181 KA Elsloo (NL); SONKE, Theodorus, NL-6143 BK Guttecoven (NL); VERZIJL, Gerardus, Karel, Maria, NL-5855 AR Well (NL); WIERTZ, Roel, Wim, NL-6441 LC Brunssum (NL)
(74) Representative: Hoogendam, Gerrie Christine
(86) International application number: PCT/EP2006/010089
(87) International publication number: WO 2007/045470

(56) References cited:
- EP-A- 0 456 138
- WO-A-95/30740
- WO-A-98/49133
- LIU C F ET AL: "Subtilisin-catalyzed synthesis of amino acid and peptide esters. Application in a two-step enzymatic ligation strategy." ORGANIC LETTERS. 27 DEC 2001, vol. 3, no. 26, 27 December 2001 (2001-12-27), pages 4157-4159, XP002376276 ISSN: 1523-7060

## Description

The invention relates to a process for the preparation of an optionally N-protected oligopeptide C-terminal alkylester. The invention also relates to a process for the preparation of oligopeptides.

Oligopeptides have many applications, for instance as pharmaceutical, food or feed ingredient or agrochemical.

For purpose of this invention, with peptides is meant any chain of two or more amino acids. For purpose of this invention, with oligopeptides is meant any linear chain of 2-100 amino acids.

Enzymatic oligopeptide synthesis, which is defined for the purpose of the invention as the synthesis of oligopeptides in which peptidic bonds are formed by an enzymatic coupling reaction, has several advantages over chemical oligopeptide synthesis. For instance, the cost-price in case of large scale production is lower due to the fact that no or limited amino acid side chain protection is required. Also, the process is less environmentally unfriendly since no additional activating agents and less organic solvents are required. Furthermore, enzyme-catalyzed couplings are devoid of racemization (according to N. Sewald and H.-D. Jakubke, in: "Peptides: Chemistry and Biology", 1st reprint, Ed. Wiley-VCH Verlag GmbH, Weinheim 2002, section 4.6.2, p 250) leading to more pure products and/or easier isolation.

With respect to the enzymatic coupling method there are two options to generate the peptidic bond. In the so-called thermodynamic (or equilibrium-controlled) approach, the carboxy component bears a free carboxylic acid functionality, while in the kinetically controlled approach an activated carboxy component is used, preferably in the form of an alkylester, for example in the form of a methylester. The thermodynamic approach has 3 major disadvantages: i) the equilibrium is usually on the side of peptide bond cleavage so that the coupling yields are poor; ii) a large amount of enzyme is usually required; iii) the reaction rates are usually very low. In the kinetically controlled approach alkyl esters are required as starting material but much less enzyme is required, the reaction time is significantly shorter, and, above all, the maximum obtainable yields are usually considerably higher. Therefore, for industrial application, an enzymatic oligopeptide synthesis concept based on a kinetic approach, i.e. using an activated carboxy component, is most attractive (according to N. Sewald and H.-D. Jakubke, in: "Peptides: Chemistry and Biology", 1st reprint, Ed. Wiley-VCH Verlag GmbH, Weinheim 2002, section 4.6.2).

Enzymatic peptidic bond synthesis can be performed in the C→N terminal direction or in the N→C terminal direction.

An example of enzymatic oligopeptide synthesis in the C→N terminal direction is given in Scheme 1. Scheme 1 is used as an example for obtaining a tripeptide and is not meant to limit the invention in any way. 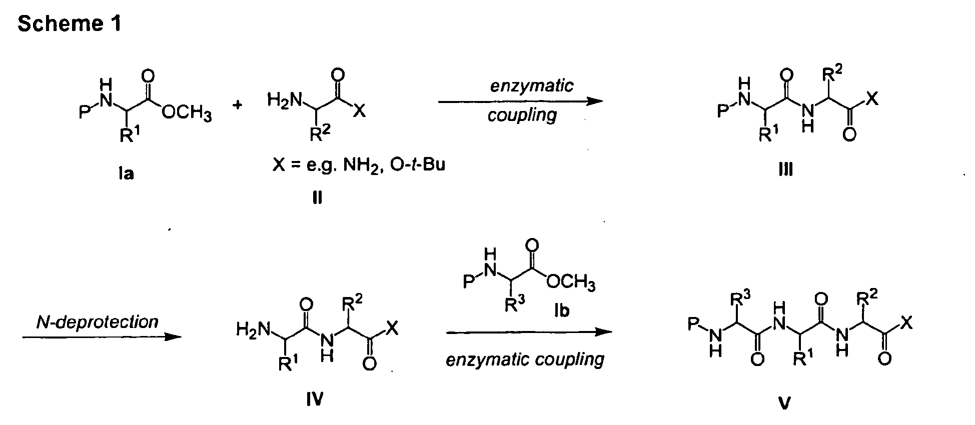 In Scheme 1, P stands for an N-terminal protecting group. R¹, R² and R³ stand for an amino acid side chain. As is indicated by Scheme 1, the enzymatic synthesis in the C→N terminal direction starts with an enzymatic coupling of a C-protected amino acid of formula **II** to an N-protected amino acid of formula I**a,** the latter being C-activated, in this case by a methyl ester. The formed dipeptide of formula **III** may then be N-deprotected and the resulting dipeptide of the formula **IV,** bearing a free amino function, may subsequently be coupled enzymatically to another N-protected (and C-activated) amino acid building block of formula **Ib** resulting in the formation of a tripeptide of formula **V.** This N-deprotection and coupling cycle may be repeated until the desired oligopeptide sequence is obtained, after which the N- and C-protecting groups may be removed to give the desired (unprotected) oligopeptide. It is also possible to couple the N-protected amino acid of formula **Ia** with a C-terminal protected oligopeptide instead of with the C-terminal protected amino acid of formula **II.**

A main disadvantage of the synthesis of oligopeptides in the C→N direction is that the N-protecting group is removed after each cycle to allow addition of a further N-protected amino acid residue. The introduction of an (expensive) N-protecting group on an amino acid (ester) and the removal and usually disposal of this same (expensive) N-protecting group after the peptide coupling, make synthesis of oligopeptides in the C→N direction unattractive from a practical and economical point of view.

An example of enzymatic oligopeptide synthesis in the N→C terminal direction is given in Scheme 2. Scheme 2 is used as an example for obtaining a tripeptide and is not meant to limit the invention in any way. 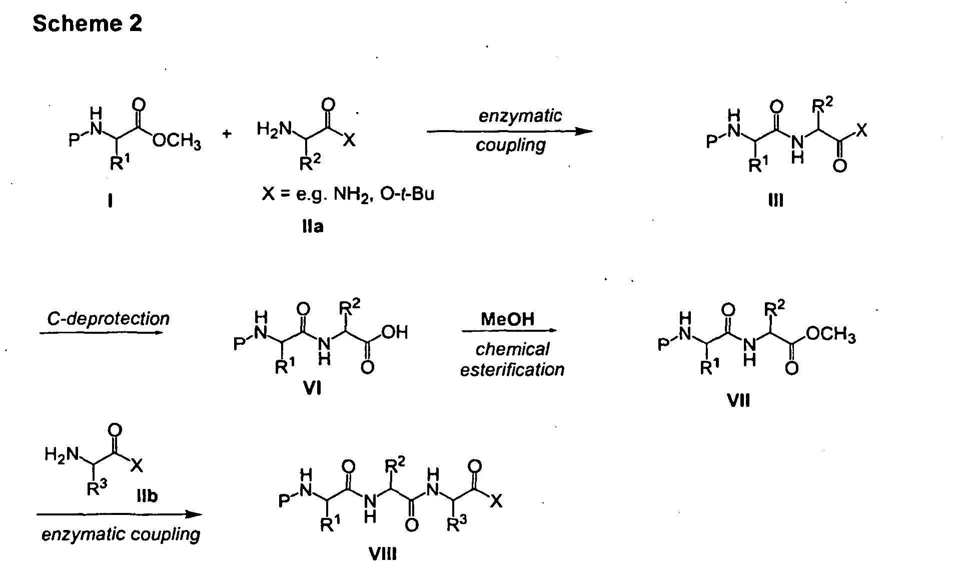 In Scheme 2, P stands for an N-terminal protecting group. R¹, R² and R³ stand for an amino acid side chain. As is indicated in Scheme 2, enzymatic synthesis in the N→C terminal direction also starts with an enzymatic coupling of a C-protected amino acid of formula **IIa** with an N-protected amino acid of formula **I,** the latter compound being C-activated, in this case by a methyl ester. The formed dipeptide of formula **III** may then be C-deprotected. The resulting dipeptide of formula **VI,** bearing a free carboxylic acid function, may then be "reactivated" to form an N-protected oligopeptide alkylester of the formula **VII,** in the case of Scheme 2 a methyl ester. This esterification is typically executed by a chemical transformation using an alcohol (for instance methanol) and a reagent such as sulphuric acid or thionyl chloride. The N-protected oligopeptide. alkylester of formula **VII** may subsequently be coupled with another C-protected amino acid of formula **IIb** which will result in the formation of a tripeptide of formula **VIII.** This C-deprotection and coupling cycle may be repeated until the desired amino acid sequence is obtained, after which the N- and C-protective groups may be removed to give the desired (unprotected) oligopeptide. It is also possible to couple the N-protected amino acid of formula **I** with a C-terminal protected oligopeptide instead of with the C-terminal protected amino acid of formula **IIa.**

Synthesis of oligopeptides in the N→C direction does not require the repeated addition and removal of an (expensive) N-protecting group. However, in order to allow the addition of a further amino acid residue, two reaction steps are needed: the C-terminus needs to be deprotected, after which it should be activated, for instance by esterification of the formed carboxylic acid group. Therefore, in total three reaction steps (deprotection, activation and coupling) are needed for the addition of one amino acid residue to an N-protected oligopeptide for oligopeptide synthesis in the N→C direction.

Suitable C-protective groups for the synthesis of oligopeptides in the N→C direction are known to the person skilled in the art. An example of a suitable C-protective group includes tert-butyl, which can for example be cleaved off using strongly acidic non-aqueous conditions, for instance by trifluoroacetic acid. It is advantageous to use carboxyamide as a C-terminal protective group (X = NH₂ in Scheme 2). With carboxyamide as a protective group the amino acid building blocks of formula **IIa** may for example be prepared by methylesterification of the amino acid followed by an amidation with ammonia in a 1-pot process, which preparation is simple and cost-effective. Carboxyamide cleavage by conventional means, using an aqueous solution of a strong mineral acid, causes simultaneous partial cleavage of peptidic bonds. However, selective deprotection of a carboxyamide protected C-terminus of an oligopeptide without cleaving peptidic bonds may be done enzymatically. EP 0 456 138 and EP 0 759 066 disclose an enzymatic process using a peptide amidase from the flavedo of oranges (referred to as "PAF") or from *Xanthomonas (Stenotrophomonas) maltophilia* (referred to as "PAM"), respectively, wherein the carboxyamide group of an N-(un)protected dipeptide C-terminal carboxyamide is hydrolysed to form the corresponding C-terminal carboxylic acid, whereby the peptidic bond of the dipeptide is left intact.

The document Liu, C.F. & Tam, J.P.: "Subtilisin-Catalyzed Synthesis of Amino Acid and Peptide Esters. Application in a Two-Step Enzymatic Ligation Strategy" (ORGANIC LETTERS, vol. 3, no. 26, pages 4157-4159, 27 December 2001) discloses a process for the preparation of Boc-protected or unprotected oligopeptide alkyl esters comprising the step of reacting the corresponding oligopeptides with an alkyl alcohol in the presence of the serine endopeptidase Subtilisin.

A disadvantage of the process as described in EP 0 456 138 and EP 0 759 066, however, is that separate esterification of the formed corresponding carboxylic acid - in order to activate the carboxylic acid group - is required for each further elongation of the oligopeptide chain with an amino acid. Another disadvantage is that this esterification of the carboxylic acid using a reagent such as sulphuric acid or thionyl chloride requires essentially non-aqueous conditions, whereas the enzymatic deprotection reaction of the C-terminal carboxyamide is performed in aqueous solution. Thus, extensive extraction and drying operations are required.

It is therefore an object of the invention to provide an easier process for the activation of a carboxyamide group of an optionally N-protected oligopeptide C-terminal carboxyamide into an alkylester function, which may then subsequently be used in a peptide coupling reaction.

This object is achieved by a process wherein an optionally N-protected oligopeptide alkyl ester can be directly obtained from the corresponding optionally N-protected-oligopeptide C-terminal carboxyamide.

Therefore, in a first aspect, the invention relates to a process for the preparation of an optionally N-protected oligopeptide alkylester comprising the step of b) reacting the corresponding optionally N-protected oligopeptide C-terminal carboxyamide with an alkyl alcohol in the presence of a peptide amidase. Hence, the process of the invention provides a one step process for the activation of a carboxyamide group of an N-protected oligopeptide C-terminal carboxyamide into an alkylester function, which process is simple and cost-effective.

The present invention surprisingly shows that such a peptide amidase is able to catalyse the direct conversion of an optionally N-protected oligopeptide C-terminal carboxyamide into an optionally N-protected oligopeptide C-terminal alkylester. This is surprising in view of Cerovsky, V. and M.-R. Kula, 1998, Angew. Chem. Int. Ed., pp. 1885-1887, who write that peptide amidase has a lack of esterase activity, which means that it is surprising that the enzyme can produce esters.

The optionally N-protected oligopeptide C-terminal carboxyamide is reacted with an enzyme displaying peptide amidase activity, called peptide amidase throughout the description of the invention. Peptide amidases have been described, in for instance EP 0 456 138 and EP 0 759 066, for their capability to hydrolyse the C-terminal carboxyamide group of optionally N-protected oligopeptide C-terminal carboxyamides without hydrolysing one or several of the peptidic bonds of the oligopeptide. Preferably, the peptide amidase from (the flavedo of) citrus fruit, more preferably from (the flavedo of) oranges, is used.

The peptide amidase may be used in any form. For example, the peptide amidase may be used - for example in the form of a dispersion, emulsion, a solution or in immobilized form - as crude enzyme, as a commercially available enzyme, as an enzyme further purified from a commercially available preparation, as an enzyme obtained from its source by a combination of known purification methods, in whole (optionally permeabilized and/or immobilized) cells that naturally or through genetic modification possess peptide amidase activity, or in a lysate of cells with such activity.

It will be clear to the average person skilled in the art that use can also be made of mutants of naturally occurring (wild-type) enzymes with peptide amidase activity in the process according to the invention. Mutants of wild-type enzymes can for example be made by modifying the DNA encoding the wild-type enzymes using mutagenesis techniques known to the person skilled in the art (random mutagenesis, site-directed mutagenesis, directed evolution, gene shuffling, etc.) so that the DNA encodes an enzyme that differs by at least one amino acid from the wild-type enzyme or so that it encodes an enzyme that is shorter or longer compared to the wild-type and by effecting the expression of the thus modified DNA in a suitable (host) cell. Mutants of the peptide amidase may have improved properties with respect to selectivity towards the optionally N-protected oligopeptide C-terminal alkylester and/or activity and/or stability and/or solvent resistance and/or pH prophile and/or temperature prophile and/or substrate prophile.

If the peptide amidase is not used in pure form, preferably other enzymes having peptidic bond cleavage activity are removed such that, when more than 99% of the optionally N-protected oligopeptide C-terminal carboxyamide is converted, not more than 1%, more preferably not more than 0.1 % of the peptidic bonds are cleaved. Preferably, the enzyme is used in a purified form, for example in the form as commercially available.

Alternatively, if the peptide amidase is not used in pure form, compounds that inhibit peptidic bond cleavage activity may be used to prevent peptidic bond cleavage.

In the process of the present invention, the hydrolytic activity (conversion of the optionally N-protected oligopeptide C-terminal carboxyamide into the corresponding C-terminal carboxylic acid instead of into the corresponding C-terminal alkylester) of the peptide amidase should preferably be kept as low as possible. To this end, the reaction with the peptide amidase is done in a medium that is substantially free of water. The skilled person will understand that substantially free of water means that only such an amount of water is present in the reaction medium to enable the enzyme to properly perform its catalytic activity. Preferably, the reaction medium contains less than 50 vol% water, more preferably less than 30 vol% water, most preferably less than 20 vol% water. Preferably, the reaction medium contains at least 0.1 vol%, more preferably at least 0.3 vol%, more preferably at least 0.5 vol%, more preferably at least 1 vol%, most preferably at least 5 vol% water. Preferred water concentrations in the reaction medium are between 0.5 and 50 vol%, more preferably between 1 and 30 vol%, most preferably between 5 and 20 vol%.

In one embodiment of the invention, at least part of the NH₃ liberated during the alkylesterification, is removed from the reaction mixture. Preferably, at least 50%, more preferably at least 75%, most preferably substantially all liberated NH₃ is removed from the reaction mixture. Removal of NH₃ from the reaction mixture may for example be done by adding a compound that complexates with NH₃, for instance a compound that precipitates after complexating NH₃ examples of which include MgHPO₄, Al₂O₃ and K₂SO₄. Alternatively, removal of NH₃ may for example be performed by adding an adsorbant, for instance a zeolite to the reaction mixture. Removal.of NH₃ may for example also be performed by applying an acid-base reaction thereby protonating the NH₃ or for example by evaporating the NH₃ from the reaction mixture during the reaction by applying a low pressure and/or by heating. In a preferred embodiment of the invention an NH₃ complexating agent is used that precipitates after complexation, more in particular MgHPO₄, Al₂O₃ or K₂SO₄ are used.

The optionally N-protected oligopeptide C-terminal alkylester may for example be represented by a compound of formula **X** 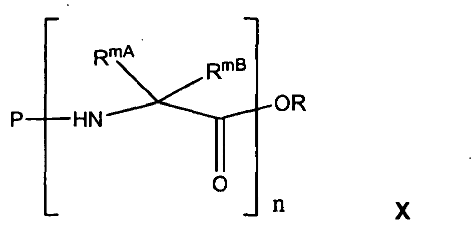 wherein P stands for H or for an N-terminal protecting group, wherein n is an integer of at least 2, wherein m stands for all integers of at least 1 but is not more than n, and
wherein R^{mA} and R^{mB} each independently stand for H, or for an amino acid side chain and wherein R stands for an optionally substituted alkyl group.

If the optionally N-protected oligopeptide C-terminal alkylester is a compound of formula **X,** the corresponding optionally N-protected oligopeptide C-terminal carboxyamide is represented by a compound of formula **IX** 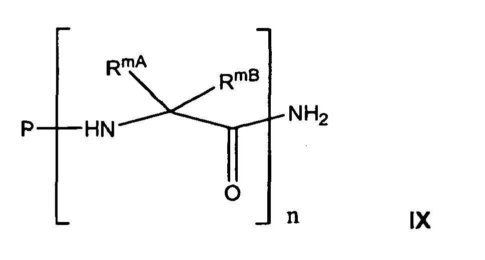 wherein P stands for H or for an N-terminal protecting group, wherein n is an integer of at least 2, wherein m stands for all integers of at least 1 but is not more than n, and wherein R^{mA} and R^{mB} each independently stand for H or for an amino acid side chain.

R preferably stands for an optionally substituted alkyl group with 1-6 C-atoms, more preferably for an optionally substituted alkyl group with 1-3 C-atoms, most preferably R stands for methyl.
Examples of non-substituted alkyl groups are: methyl, ethyl, isobutyl and n-octyl. Examples of substituted alkyl groups are: carbamoylmethyl, N-methylcarbamoylmethyl, benzyl, p-nitrobenzyl, cyanomethyl, 2,2,2-trifluoroethyl, 2,2,2-trichloroethyl and 4-pyridylmethyl.

Which alkyl alcohol to use in the process of the present invention depends on which optionally N-protected oligopeptide C-terminal alkylester is desired. For example if methanol is used, the formed optionally N-protected oligopeptide C-terminal alkylester will be the optionally N-protected oligopeptide C-terminal methylester. For example, if ethanol is used the formed optionally N-protected oligopeptide C-terminal alkylester will be the optionally N-protected oligopeptide C-terminal ethylester etc. Preferably, the alkyl alcohol is methanol.

Optimal alkyl alcohol concentrations can be determined through routine experimentation by the person skilled in the art. In a preferred embodiment of the invention, the alkyl alcohol used is methanol.

However, the presence of one or more other solvents besides the alkyl alcohol is also possible and in some cases the presence of one or more other solvents may even be advantageous, for instance to solubilize the optionally N-protected oligopeptide C-terminal carboxyamide of formula **IX.**

A surprising aspect of the process of the present invention is that the peptide amidase activity manifests itself even at high methanol concentrations, even though this is a condition under which enzymes usually display a negligible activity [K. Drauz and H. Waldmann, in: "Enzyme Catalysis in Organic Synthesis", Volume 1, 2nd Edition, Ed. Wiley-VCH Verlag GmbH, Weinheim 2002, sections 1.6 and 8.6; M. Pogorevc, H. Stecher and K. Faber in Biotechnology Letters 2002, 24, 857-860].

Preferably, the peptide amidase is added to the reaction medium in more than one portion over time, instead of in one portion at the beginning of the process

In order to prevent reaction of the free N-terminal amino function of the oligopeptide C-terminal alkylester during the peptide coupling reactions, a suitable protecting group may preferably protect this amino function. Suitable N-protecting groups are those N-protecting groups which can be used for the synthesis of (oligo)peptides and are known to the person skilled in the art. Examples of suitable N-protecting groups include Z (benzyloxycarbonyl), Boc (tert-butyloxycarbonyl) and PhAc (phenacetyl), the latter of which may be introduced and cleaved enzymatically using the enzyme PenG acylase.

In the context of the invention with 'amino acid side chain' is meant any proteinogenic or non-proteinogenic amino acid side chain. The reactive groups in the amino acid side chains may be protected by amino acid side chain protecting groups or may be unprotected. Examples of proteinogenic amino acids include: alanine, valine, leucine, isoleucine, serine, threonine, methionine, cysteine, asparagine, glutamine, tyrosine, tryptophan, glycine, aspartic acid, glutamic acid, histidine, lysine, arginine and phenylalanine. Examples of non-proteinogenic amino acids include phenylglycine and 4-fluoro-phenylalanine.

n stands for an integer of at least 2, so stands for 2, 3, 4, 5, 6, 7, 8, 9, 10, etc. m stands for all integers of at least 1 but is not more than n. In other words m represents the position of the amino acid residue in the oligopeptide chain of which the side groups R^{A} and R^{B} are part. Therefore R^{mA} represents a complete collection of n R^{A}-groups, each group potentially representing a different side group (i.e. H or an amino acid side chain) and R^{mB} represents a complete collection of n R^{B}-groups, each group potentially representing a different side group (i.e. H or an amino acid side chain).

In principle the pH used is not critical and may for example be chosen between 5 and 11, preferably between 6 and 9. The pH may vary during the reaction, but may also be kept constant by using a buffered aqueous solution using a buffer concentration of for example between 10 mM and 500 mM. Alternatively, the pH of the reaction may be kept constant by using an automated pH-stat system. Optimal pH conditions can easily be identified by a person skilled in the art through routine experimentation.

In principle the temperature used is not critical and temperatures of preferably between 0 and 45°C, more preferably between 15 and 40°C may be used. Alternatively, if a thermophilic peptide amidase is used, the temperature may be chosen higher, for example between 40 and 90°C. Optimal temperature conditions can easily be identified by a person skilled in the art through routine experimentation.

In order to isolate the optionally N-protected oligopeptide C-terminal alkylester, for example the oligopeptide of formula **X,** in a sufficiently pure form, it may sometimes be advantageous to almost completely or completely convert the corresponding optionally N-protected oligopeptide C-terminal carboxyamide, for example the C-terminal carboxyamide of formula **IX** into the C-terminal alkylester of formula **X** and partly into the corresponding C-terminal carboxylic acid. The C-terminal carboxylic acid is a result of the hydrolytic side reaction due to the presence of water required for the peptide amidase activity. This (almost) complete conversion of the optionally N-protected oligopeptide C-terminal carboxyamide may even be advantageous in cases where the total amount of optionally N-protected oligopeptide C-terminal alkylester at full conversion of the corresponding C-terminal carboxyamide is lower than at only partial conversion, since the optionally N-protected oligopeptide C-terminal alkylester is usually more difficult to separate from the corresponding C-terminal carboxyamide than from the corresponding C-terminal carboxylic acid. The optionally N-protected oligopeptide C-terminal alkylester can for example be separated from the corresponding C-terminal carboxylic acid using a two-phase system with a water-immiscible organic solvent and an aqueous phase having a pH value above the pKa value of the free carboxyl function of the C-terminal carboxylic acid (usually above approximately 3.5); in this case the optionally N-protected oligopeptide C-terminal alkylester remains in the organic phase, while the corresponding C-terminal carboxylic acid is removed to the aqueous phase in one or more extractions.

In a second aspect, the present invention provides a process for the preparation of an oligopeptide comprising the process according to the invention.

In particular, the present invention provides a process for the preparation of an oligopeptide comprising the following steps:
b) reacting an optionally N-protected oligopeptide C-terminal carboxyamide with an alkyl alcohol in the presence of a peptide amidase according to the first aspect of the invention,
c) reacting the formed optionally N-protected oligopeptide C-terminal alkylester with an amino acid C-terminal carboxyamide or with an oligopeptide C-terminal carboxyamide in the presence of an enzyme that catalyzes peptide bond formation to form an optionally N-protected oligopeptide C-terminal carboxyamide.

Accordingly, the present invention also provides a process for the synthesis of oligopeptides that allows oligopeptide synthesis in the N→C direction using less reaction steps than previously, since the C-terminus is deprotected and simultaneously activated by enzymatic means. Additionally, no extensive extraction and dehydration procedures are required for the C-terminal carboxylic acid intermediate. This advantageously allows a completely enzymatic process for the synthesis of peptides in the N→C direction, since also both the oligopeptide chain elongation and the protection and deprotection of the N-terminal amino function may be performed completely enzymatically. Therefore, the process of the invention is more attractive than the previously known processes, for instance from an economical and/or an environmental point of view.

With regard to step c) any enzyme can be used that can catalyze peptide bond formation. Such enzymes are known to the skilled person and examples include proteases, acylases, for instance penG acylases and amino acid ester hydrolases.

Steps b) and c) may be repeated until the optionally N-protected oligopeptide C-terminal carboxyamide with the desired amino acid sequence is obtained.

Therefore, the invention also relates to a process for the synthesis of an oligopeptide comprising the following steps:
b) reacting an optionally N-protected oligopeptide C-terminal carboxyamide with an alkyl alcohol in the presence of a peptide amidase according to the first aspect of the invention,
c) reacting the formed optionally N-protected oligopeptide C-terminal alkylester with an amino acid C-terminal carboxyamide or with an oligopeptide C-terminal carboxyamide in the presence of an enzyme that catalyzes peptide bond formation to form an optionally N-protected oligopeptide C-terminal carboxyamide, wherein step b) and c) are repeated until an optionally N-protected oligopeptide C-terminal carboxyamide of the desired amino acid sequence is obtained.

The optionally N-protected oligopeptide C-terminal carboxyamide, used as starting material in step b), may be prepared by a) reacting an optionally N-terminal protected amino acid C-terminal alkylester or an optionally N-terminal protected oligopeptide C-terminal alkylester with an amino acid C-terminal carboxyamide or with an oligopeptide C-terminal carboxyamide in the presence of an enzyme that catalyzes peptide bond formation.

Therefore, the invention also relates to a process for the preparation of an oligopeptide comprising the following steps:
a) reacting an optionally N-terminal protected amino acid C-terminal alkylester or an optionally N-terminal protected oligopeptide C-terminal alkylester with an amino acid C-terminal carboxyamide or with an oligopeptide C-terminal carboxyamide in the presence of an enzyme that catalyzes peptide bond formation,
b) reacting the formed optionally N-protected oligopeptide C-terminal carboxyamide with an alkyl alcohol in the presence of a peptide amidase according to the first aspect of the invention,
c) reacting the formed optionally N-protected oligopeptide C-terminal alkylester with an amino acid C-terminal carboxyamide or with an oligopeptide C-terminal carboxyamide in the presence of an enzyme that catalyzes peptide bond formation to form an optionally N-protected oligopeptide C-terminal carboxyamide,
wherein step b) and c) may be repeated until the optionally N-protected oligopeptide C-terminal carboxyamide of the desired amino acid sequence is obtained.

If desired, the optionally N-protected oligopeptide C-terminal carboxyamide of the desired amino acid sequence may be deprotected on the C-terminus and/or on the N-terminus and/or - if at least one amino acid side chain protecting group is present - the optionally N-protected oligopeptide C-terminal carboxyamide may be deprotected on at least one of the amino acid side chains, after which the protected or unprotected oligopeptide may be recovered.

Deprotection of the N-terminal protecting group of the formed N-protected oligopeptide C-terminal carboxyamide or N-protected C-terminal deprotected oligopeptide may be performed by methods known to the person skilled in the art. Preferably, the N-terminal protecting group is enzymatically removed, more preferably the N-terminal protective group is both introduced and removed enzymatically.

Enzymatic introduction and/or removal of the N-protecting group is particularly advantageous, since this renders the process more cost-effective and environmentally friendlier. This novel combination of i) an enzymatically introducable and cleavable N-protecting group and ii) a stepwise enzymatic elongation of the peptide chain with amino acid C-terminal carboxyamides or oligopeptide C-terminal carboxyamides and iii) a repetitive enzymatic C-terminal carboxyamide deprotection and simultaneous activation into the C-terminal alkylester of the growing oligopeptide chain, allows for the concept of a completely enzymatic synthesis of oligopeptides which has never been described before.

Enzymes capable of introducing and removing an N-terminal protective group of an oligopeptide C-terminal carboxyamide or alkylester are known to the skilled person and examples of such enzymes include penG acylases.

Conventional means to remove the C-terminal carboxyamide function, using an aqueous solution of a strong mineral acid, causes simultaneous partial cleavage of peptidic bonds. Therefore, deprotection of the C-terminus of the formed optionally N-protected oligopeptide C-terminal carboxyamide may preferably be performed by using a peptide amidase under conditions in which the corresponding C-terminal alkylester is formed to a limited extent or not at all, for example in an aqueous solution containing not more than 40 wt% alkyl alcohol, more preferably not more than 10 wt% alkyl alcohol, most preferably not more than 3 wt% alkyl alcohol.

If an N-protected oligopeptide C-terminal carboxyamide without or with one or several amino acid side chain protecting groups, as obtained after the last peptide coupling step, is the desired end-product, this can be directly recovered, for example using extraction or crystallization methods known to the person skilled in the art. In case a partially protected oligopeptide is the desired end product, the C- and/or N- and/or - if one or several amino acid side chain protecting groups are present after the last peptide coupling step - amino acid side chain deprotection reaction(s) can be carried out and, optionally after a work-up procedure, the desired partially protected end product can be recovered. Usually, however, the completely deprotected oligopeptide is the desired end-product which can be obtained by consecutive N-, C- and - if one or several amino acid side chain protecting groups are present after the last peptide coupling step - amino acid side chain deprotection steps. Procedures to finally recover the completely deprotected oligopeptide, for instance including extraction(s) and crystallization(s) can be identified by a person skilled in the art.

Preferably, the reaction mixture is worked up after the coupling reaction of step a) and/or c) to remove any remaining starting compound(s) and any side-product(s) that may have been formed. A particularly useful embodiment is to react the optionally N- protected amino acid C-terminal alkylester or the optionally N-protected oligopeptide C-terminal alkylester with a (limited) excess of the amino acid C-terminal carboxyamide or the oligopeptide C-terminal carboxyamide to reach full or almost full conversion of the ester compound and to subsequently partition the reaction mixture in a two-phase system consisting of a water-immiscible organic solvent and an aqueous phase having a pH value which is lower than the pKa of the amino acid C-terminal carboxyamide or oligopeptide C-terminal carboxyamide. In such a case the excess amino acid C-terminal carboxyamide or oligopeptide C-terminal carboxyamide can be extracted into the aqueous phase in the protonated form with the desired coupling product remaining in the organic phase. It may be advantageous to apply multiple aqueous extractions or back-extractions of the aqueous phase with an organic solvent in order to optimize the yield and/or purity of the N-protected oligopeptide C-terminal carboxyamide coupling product. The most suitable conditions can be easily determined by a person skilled in the art.

Examples of oligopeptides that may be prepared using the process of the present invention are the tripeptides H-Gly-Tyr-Phe-OH and H-Arg-Gly-Asp-OH and the tetrapeptide H-Tyr-D-Ala-parafluoro-Phe-Phe-NH₂. The oligopeptides produced by the process of the invention preferably are a linear chain of 2-50 amino acids, more preferably a linear chain of 2-20 amino acids, most preferably a linear chain of 2-10 amino acids.

### Examples

### Materials

Peptide Amidase from Flavedo (referred to as "PAF") was obtained from Fluka and used as such (activity: 2.4 U/mL in the deamidation of Z-Gly-Tyr-NH₂ to Z-Gly-Tyr-OH). In case freeze-dried PAF was used, 5 mL of the commercial PAF solution was freeze-dried using a Sentry Freeze-Mobile 125 (G-25) giving 0.40 g solid PAF having an activity of 30 U/g and containing 2.5 wt% water, as determined by Karl-Fischer titration. The microbial Peptide Amidase (referred to as "PAM") from *Stenotrophomonas* (formerly known as *"Xanthomonas') maltophilia* was obtained from Jülich Fine Chemicals (Jülich, Germany) and used as such (activity: 2.87 U/mL in the deamidation of Z-Gly-Tyr-NH₂ to Z-Gly-Tyr-OH). The enzymatic activity of PAF and PAM was assayed at 30 °C by hydrolysis of Z-Gly-Tyr-NH₂ in 50 mM aq.Tris/HCl buffer (pH = 7.5) containing 5 vol% DMF by monitoring the conversion to Z-Gly-Tyr-OH using the HPLC method as described in Example 1A.

Alcalase was obtained from Novozymes (batch PLN 04810) and dried before use. Drying was performed by suspending 8.0 mL alcalase solution in 60 mL ethanol and centrifuging the suspension at 3000G at 4 °C. The supernatant was decanted and the solid 3 x more suspended in 60 mL ethanol and centrifuged; the resulting residue was used as such. Assemblase was obtained from DSM Anti-infectives (Delft, The Netherlands) and corresponds to penG acylase from *E. coli* covalently immobilized on a polymer support according to patent WO 97/04086. Separase was obtained from DSM Anti-infectives and corresponds to penG acylase from *A. faecalis* covalently immobilized on a polymer support according to WO97/04086.

Z-Gly-Tyr-NH₂ and Z-Gly-Tyr-OH (Bachem), methanol (Fluka), phenylacetic acid and MgHPO₄ (Acros) and chlorosulphonic acid (Merck) were used as received. *t*-Butanol (Merck) was dried by distillation before use.

Z-Gly-Tyr-OMe reference material was prepared from Z-Gly-Tyr-OH according to the method as described in EP977726 (1998, by P.J.L.M. Quaedflieg and W.H.J. Boesten): chlorosulphonic acid (0.55 g, 4.72 mmol, 1.1 equiv.) was added dropwise under vigorous stirring under nitrogen to a solution of Z-Gly-Tyr-OH (1.60 g, 4.30 mmol) in methanol (14 mL) at 0-5 °C. After 2 h stirring at 50 °C the solution was cooled to 20 °C, added dropwise to 50 mL vigorously stirred 7 wt% aq. KHCO₃ and extracted with ethyl acetate (3 x 50 mL). The combined organic phase was dried (Na₂SO₄) and concentrated in vacuo giving >99 wt% pure Z-Gly-Tyr-OCH₃ in 85% yield based on Z-Gly-Tyr-OH.

¹H NMR spectra were recorded at 300 MHz in DMSO-*d*₆ on a Bruker 300 MHz Ultrashield^{™} NMR spectrometer.

In the context of the present invention 1 U corresponds to the amount of enzyme which catalyzes the formation of 1 micromol Z-Gly-Tyr-OH per minute in aqueous solution at pH 7.5 and 30°C using Z-Gly-Tyr-NH₂ as substrate.

### Example 1: Enzymatic conversion of Z-Gly-Tyr-NH₂ (1) to Z-Gly-Tyr-OMe (2)

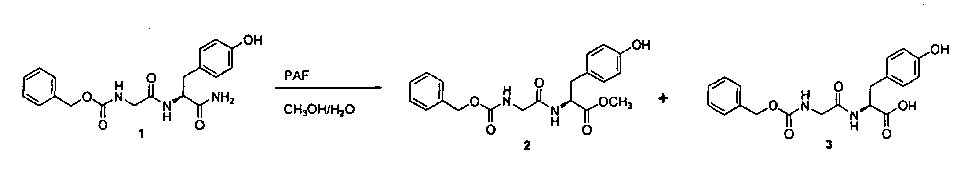

### Example 1A: Conversion of Z-Gly-Tyr-NH₂ (1) using repetitive addition of PAF

To a solution of **1** (15 mg, 0.04 mmol) in 3.2 g MeOH was added 250 µL of Peptide Amidase from Flavedo (PAF; 2.4 U/mL) and MgHPO₄ (35 mg, 0.2 mmol, 5 eq) at 30 °C. The reaction mixture was stirred for 186 h at this temperature and monitored with HPLC (see below) while at intervals of 24 h a fresh amount of PAF (250 µL) was added. The course of the conversion is shown in Table 1. After 66 h the conversion of amide **1** to ester **2** was maximal (41%). At that time 10% of the acid **3** had been formed as well.

HPLC analysis: the conversion of **1** to **2** and **3** was monitored with reversed-phase HPLC on an Prevail column (250 x 4.6 mm I.D., 5 µm) from Alltech using a gradient of 0.1% aq. HCOOH and acetonitrile (1.0 mL/min, at 40°C). At t=0, 15 vol% acetonitrile was used which was increased to 74.5 vol% during 8 min and was kept at 74.5 vol% for 7 min. At 15.1 min the gradient prophile returned to starting conditions. Total analysis time was 22 min. The injection volume was 5 µL and detection was performed using a spectrophotometer at UV 254 and 270 nm. Retention times for **1, 3** and **2** were 5.9, 9.8 and 25.4 min, respectively.

**Table 1. Conversion of Z-Gly-Tyr-NH₂ using repetitive addition of PAF**

| **Time (h)** | **Z-Gly-Tyr-NH₂ (1) (mol%)** | **Z-Gly-Tyr-OMe (2) (mol%)** | **Z-Gly-Tyr-OH (3) (mol%)** | **PAF added (µL)** | **H₂O (wt%)** |
|---|---|---|---|---|---|
| 0 | 100 | 0 | 0 | 250 | 7 |
| 18 | 84 | 16 | 1 | 250 | 13 |
| 42 | 69 | 28 | 3 | 250 | 18 |
| 66 | 58 | 35 | 7 | 250 | 22 |
| 138 | 50 | 41 | 10 | 250 | 26 |
| 186 | 50 | 40 | 10 | | 26 |

### Example 1B: Conversion of Z-Gly-Tyr-NH₂ (1) with all PAF added at the start

To a solution of **1** (15 mg, 0.04 mmol) in 3.2 g MeOH was added 830 µL of Peptide Amidase from Flavedo (PAF; 2.4 U/mL) and MgHPO₄ (35 mg, 0.2 mmol, 5 eq) at 30 °C. The reaction was monitored with HPLC (using the same method as described in Example 1 A) showing that at both 24 h and 40 h the conversion of 1 to ester **2** was 14% and to acid **3** 0.2%.

### Example 1C: Complete conversion of Z-Gly-Tyr-NH₂ (1) using repetitive PAF addition

To a solution of **1** (15 mg, 0.04 mmol) in 3.2 g MeOH was added 250 µL of Peptide Amidase from Flavedo (PAF; 2.4 U/mL) and MgHPO₄ (35 mg, 0.2 mmol, 5 eq) at 30 °C. The reaction mixture was stirred for 15 days at this temperature and monitored with HPLC (see Example 1A) while at intervals of 1 day a fresh amount of PAF (250 µL) was added. The course of the conversion is shown in Table 2. Evidently, after 15 days **1** had almost completely been converted to **2** (30%) and **3** (60%).

**Table 2. Conversion of Z-Gly-Tyr-NH₂ using repetitive PAF addition**

| **Time (h)** | **Z-Gly-Tyr-NH₂ (1) (mol%)** | **Z-Gly-Tyr-OMe (2) (mol%)** | **Z-Gly-Tyr-OH (3) (mol%)** | **PAF added (µL)** | **H₂O (wt%)** |
|---|---|---|---|---|---|
| 0 | 100 | 0 | 0 | 250 | 7 |
| 23 | 94 | 6 | 0 | 250 | 13 |
| 48 | 82 | 15 | 2 | 250 | 18 |
| 72 | 75 | 19 | 6 | 250 | 22 |
| 96 | 68 | 23 | 9 | 250 | 26 |
| 168 | 61 | 24 | 15 | 250 | 30 |
| 192 | 56 | 25 | 19 | 250 | 34 |
| 216 | 48 | 29 | 23 | 250 | 37 |
| 239 | 39 | 30 | 31 | 250 | 39 |
| 311 | 27 | 34 | 40 | 250 | 42 |
| 359 | 14 | 31 | 55 | 250 | 44 |
| 383 | 9 | 30 | 60 | | 46 |

### Example 2: Influence of additives

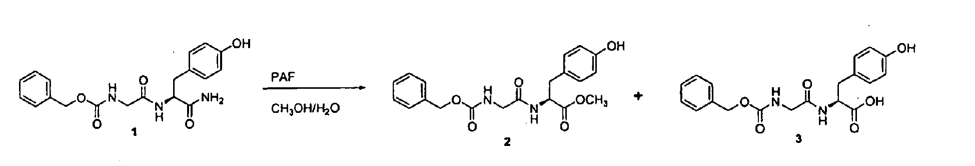

To a solution of **1** (15 mg, 0.04 mmol) in 3.16 g MeOH was added 249 µL of Peptide Amidase from Flavedo (PAF; 2.4 U/mL) and a certain amount of additive (see Table 3). After stirring the reaction mixture at 30 °C for 24 h an aliquot was withdrawn and analysed with HPLC using the same HPLC method as described in Example 1A. The amount of formed ester **2** is given in Table 3.

**Table 3. Influence of additives on Z-Gly-Tyr-NH₂ conversion with PAF.**

| **Additive** | **Amount (mg)** | **eq based on 1** | **Z-Gly-Tyr-OMe (3) (mol%)** |
|---|---|---|---|
| MgHPO₄ | 35 | 5 eq | 16 |
| Al₂O₃ | 20.4 | 5 eq | 17 |
| Trimethylorthoformiate | 21.2 | 5 eq | 7.6 |
| K₂SO₄ | 34.9 | 5eq | 7.5 |

### Example 3: Influence of the amount of water

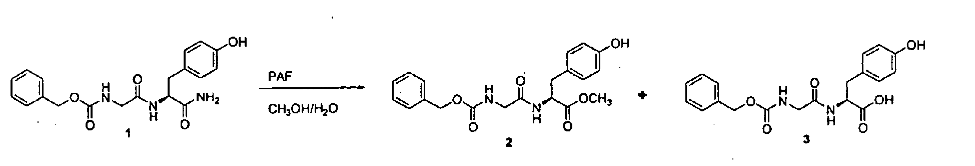

To a solution of **1** (15 mg, 0.04 mmol) in MeOH and a certain amount of demineralised water or Tris/HCl buffer (pH = 7.5, containing 5 wt% DMF) (see the table below) with a total solution volume of 4 mL, was added a certain amount (see the table below) of freeze-dried Peptide Amidase from Flavedo (PAF; 30 U/g) and, in some cases (see Table 4), subsequently 34.9 mg (0.2 mmol, 5 eq) MgHPO₄. The resulting reaction mixture was stirred at 30 °C for 24 h and an aliquot was withdrawn and analysed with HPLC using the same HPLC method as described in Example 1A. The amounts of formed ester **2** and acid **3** are given in Table 4.

**Table 4. Influence of amount of water on Z-Gly-Tyr-NH₂ conversion with PAF**

| **MgHPO₄ (yes/no)** | **MeOH (wt%)** | **demi (wt%)** | **Tris/HCl (wt%)** | **Freeze-dried PAF (mg)** | **Z-Gly-Tyr-OH 2 (mol%)** | **Z-Gly-Tyr-OCH₃ 3 (mol%)** |
|---|---|---|---|---|---|---|
| Yes | 100 | | | 50 | <0.5 | < 0.5 |
| Yes | 80 | 20 | | 20 | 3 | 8 |
| Yes | 50 | 50 | | 20 | 13 | 10 |
| Yes | 80 | 0 | 20 | 20 | 9 | 7 |
| Yes | 90 | 10 | | 20 | 2 | 4 |
| No | 95 | 5 | | 6.4 | < 0.5 | 1.4 |
| Yes | 95 | 5 | | 6.4 | < 0.5 | 1.2 |
| No | 95 | | 5 | 6.4 | < 0.5 | 0.5 |
| Yes | 95 | | 5 | 6.4 | < 0.5 | 0.6 |
| No | 95 | 5 | | 128 | < 0.5 | 4 |
| Yes | 95 | 5 | | 128 | 1 | 7 |

### Example 4: Fully enzymatic synthesis of the tripeptide H-Gly-Tyr-Phe-OH (14) using the N → C approach with PAF

For the synthesis of **14**, the synthetic scheme below was followed.

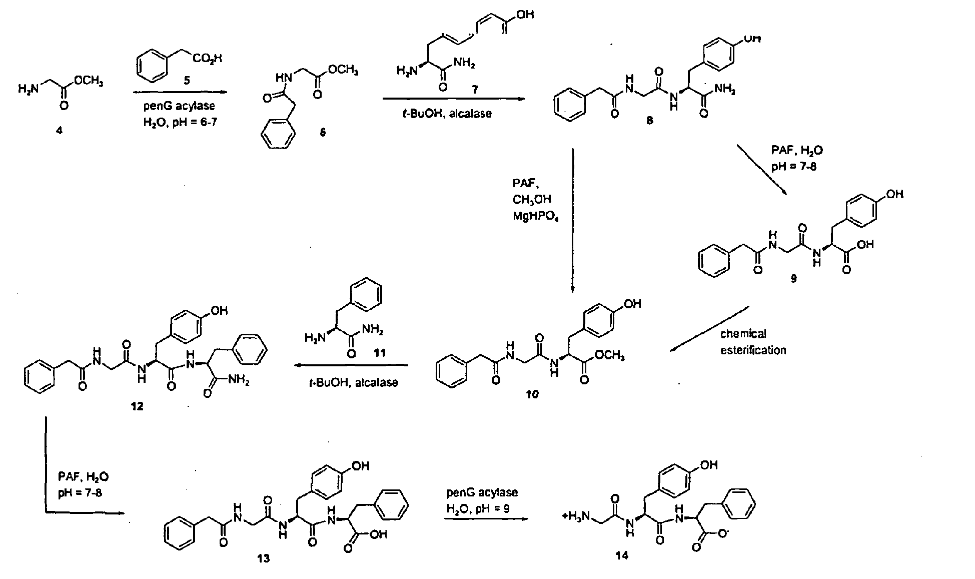

### 4A. Enzymatic N-protection of GlyOMe.HCl (4) to PhAc-Gly-OMe (6)

100.0 g GlyOMe.HCl **(4**, 0.796 mol) and 108.4 g phenylacetic acid (**5**, 0.796 mol) were suspended in 500 mL water. The pH was adjusted to 6.3 by the addition of 83.3 mL 32 wt% NaOH. To the resulting solution 48 g of immobilized assemblase was added and the reaction mixture was stirred for 16 h at 20 °C. The resulting slurry was cooled to 0 °C and stirred at that temperature for 1 h and the product PhAc-GlyOMe **(6)** and the immobilized assemblase were isolated by filtration. The solid material was slurried up in EtOAc (500 mL) and the immobilized assemblase was filtered off. The EtOAc layer was dried (Na₂SO₄) and concentrated in vacuo yielding 100.0 g (0.483 mol) of **6** (61% based on **4).**

¹H-NMR (DMSO-*d*₆), δ (ppm): 3.63 (3H, s, OCH₃); 3.69 (2H, s, CH₂ PhAc); 3.87 (2H, s, CH₂ Gly); 7.27-7.34 (5H, m, PhAc); 8.5 (1 H, bs, NH).

### 4B. Enzymatic coupling to PhAc-Gly-Tyr-NH, (8)

To a solution of 54.6 g (0.264 mol) PhAc-Gly-OMe **(6)** in 640 g *t-*butanol was added 61.8 g (0.343 mol; 1.30 equiv.) L-Tyr-NH₂**(7)**. To the resulting slurry was added dried alcalase based on 8 mL of alcalase solution and the reaction mixture was stirred at 35 °C for 160 h while at intervals of 24 h the same amount of alcalase was added. After 160 h an aliquot was taken out of the reaction mixture and analysed by HPLC (see below). The conversion of **6** to PhAc-Gly-Tyr-NH₂ **(8)** was 60% whereas the hydrolysis to the by-product PhAc-Gly-OH was 10%. The reaction mixture was concentrated in vacuo and the residue partitioned between 100 mL EtOAc and 100 mL water and the pH of the aqueous phase was adjusted to 3.0 (using aq. 12 N HCl). After vigorous stirring **8** crystallized and was isolated by filtration. After phase separation of the resulting mother liquor the EtOAc layer was stirred with another portion of 100 mL water at pH 3.0. This resulted in the crystallization of another portion of **8,** which was isolated by filtration. Of the resulting mother liquor the EtOAc layer.was stirred with another 100 mL water at pH 7.5 (adjusted with aq. 1 N NaOH) resulting in the crystallization of another portion of **8,** which was also isolated by filtration. Totally, 30.0 g (0.084 mol) **8** (32% yield based on **6)** was obtained.

¹H-NMR (DMSO-*d*₆), δ (ppm): 2.6 (1H, dd, CH₂ Tyr); 2.9 (1H, dd, CH₂ Tyr); 3.4 ( 2H, s, CH₂ PhAc); 3.6 (1 H, dd, CH₂ Gly); 3.8 (1H, dd, CH₂ Gly); 4.35 (1H, m, α-H Tyr); 6.6 (2H, d, CH Tyr); 6.9 (2H, d, CH Tyr); 7.1 (1H, s, NH₂); 7.25-7.35 (5H, m, PhAc); 7.4 (1 H, s, NH₂); 8.0 (1 H, d, NH Tyr); 8.25 (1 H, t, NH Gly); 9.3 (1 H, s, OH Tyr).

HPLC analysis: the coupling of **6** and **7** to **8** was monitored via reversed-phase HPLC on a Prevail column (250 x 4.6 mm I.D., 5 µm) from Alltech using a gradient of aq. 100 mM HClO₄ (pH 1.0) and acetonitrile (1.0 mL/min) at 40°C. At t=0 min, 25 vol% acetonitrile was used which was increased to 70 vol% during 8 min and was kept at 70 vol% for 2 min. At 10.1 min the gradient prophile returned to starting conditions. The total analysis time was **16** min. The injection volume was 5µL and detection was performed with a spectrophotometer at UV 220 nm. Retention times for 7, PhAc-Gly-OH, **8** and **6** were 2.9, 4.9, 5.3 and 5.9 min, respectively.

### 4C-1. Enzymatic conversion of PhAc-Gly-Tyr-NH₂ (8) to PhAc-Gly-Tyr-OMe (10)

To a solution of 5 g (14 mmol) PhAc-Gly-Tyr-NH₂ **(8)** in 1050 g MeOH was added 83 mL of Peptide Amidase from Flavedo (PAF; 2.4 U/mL) at 30 °C and 11.7 g (67 mmol) MgHPO₄. Every 24 h 83 mL fresh enzyme was added. The course of the reaction was monitored by HPLC (see below). After 140 h of stirring the conversion of **8** to **10** was 40% and the conversion of **8** to PhAc-Gly-Tyr-OH **(9)** by hydrolysis was 60%. The desired ester **10** could be isolated by evaporation of the methanol, partitioning of the residue between 50 mL EtOAc and 50 mL water at pH = 7, washing the EtOAc layer with another 50 mL water at pH = 7 and subsequently drying (Na₂SO₄) and concentrating the EtOAc layer in vacuo. This gave 1.9 g **10** (5.14 mmol, 37% yield based on **8).**

¹H-NMR (DMSO-*d₆*), δ (ppm): 2.6 (1 H, dd, CH₂ Tyr); 2.9 (1 H, dd, CH₂ Tyr); 3.5 (2H, s, CH₂ PhAc); 3.6 (3H, s, CH₃); 3.7 (1H, dd, CH₂ Gly); 3.9 (1H, dd, CH₂ Gly); 4.4 (1 H, m, α-H Tyr); 6.7 (2H, d, CH Tyr); 7.0 (2H, d, CH Tyr); 7.28 (5H, m, PhAc); 8.3 (1 H, d, NH Tyr); 8.3 (1 H, t, NH Gly); 9.3 (1 H, s, OH Tyr).

HPLC analysis: the conversion of **8** to **10** was monitored via the same reversed-phase HPLC method as described in Example 1A. Retention times for **8, 9** and **10** were 6.9, 7.8 and 8.9 min, respectively.

### 4C-2. Comparative example: enzymatic hydrolysis of PhAc-Gly-Tyr-NH₂ (8) to PhAc-Gly-Tyr-OH (9) followed by chemical esterification to give PhAc-Gly-Tyr-OMe (10)

To a suspension of 15 g (42 mmol) PhAc-Gly-Tyr-NH₂ **(8)** in 4.2 L50 mM aq.Tris/HCl buffer (pH = 7.5) containing 5 vol% DMF was added 87.6 mL of Peptide Amidase from Flavedo (PAF; 2.4 U/mL) and the reaction mixture was stirred for 24 h at 30 °C. An aliquot was withdrawn from the reaction mixture and analyzed by HPLC (see below) showing that the conversion of **8** to **9** was 100%. The reaction mixture was concentrated in vacuo to 500 mL and the pH was brought to 1.0 (using 32 wt% aq. HCl). After extraction with ethyl acetate (3 x 300 mL) the combined organic phase was dried (Na₂SO₄) and concentrated in vacuo yielding a residue containing 10.8 g (30 mmol) **9** (72% based on **8)** and 8.8 g (0.12 mol) DMF.

¹H-NMR (DMSO-*d*₆), δ (ppm): 2.6 (1 H, dd, CH₂ Tyr); 2.9 (1 H, dd, CH₂ Tyr); 3.4 (2H, s, CH₂ PhAc); 3.7 (1 H, dd, CH₂ Gly); 3.9 (1H, dd, CH₂ Gly); 4.4 (1 H, m, α-H Tyr); 6.7 (2H, d, CH Tyr); 7.0 (2H, d, CH Tyr); 7.31-7.33 (5H, m, PhAc); 8.1 (1H, d, NH Tyr); 8.3 (1H, t, NH Gly); 9.2 (1H, s, OH Tyr); 12 (1H, bs, COOH). HPLC analysis: the conversion of **8** to **9** was monitored via the same reversed-phase HPLC method as described in Example 1A. Retention times for **8** and **9** were 6.9 and 7.8 min, respectively.

To a cooled (0 °C) solution of the residue containing 10.8 g of **9** in 111 g methanol was added dropwise 3.84 g (33 mmol) chlorosulphonic acid (CISO₃H) and the reaction mixture was heated at 45 °C for 2 h. TLC analysis (eluent n-butanol/HCOOH/water 75/15/10 v/v/v) showed that the conversion of **9** to **10** was complete. The reaction mixture was cooled to ambient temperature, poured into a stirred mixture 50 mL 0.2 M aq. KHCO₃ and 100 mL water and extracted with ethyl acetate (2 x 200 mL). The combined organic phase was dried (Na₂SO₄) and concentrated in vacuo yielding a colorless oil. The oil was taken up in CH₂Cl₂ (100 mL), washed with water (100 mL) and the CH₂Cl₂ layer was dried (Na₂SO₄) and concentrated in vacuo yielding 9.2 g of **10** as a colorless oil (24.9 mmol, 83% yield based on **9).**

¹H-NMR (DMSO-*d*₆), δ (ppm): 2.6 (1H, dd, CH₂ Tyr); 2.9 (1H, dd, CH₂ Tyr); 3.5 (2H, s, CH₂ PhAc); 3.6 (3H, s, CH₃); 3.65 (1 H, dd, CH₂ Gly); 3.9 (1 H, dd, CH₂ Gly); 4.4 (1 H, m, α-H Tyr); 6.7 (2H, d, CH Tyr); 7.0 (2H, d, CH Tyr); 7.28 (5H, m, PhAc); 8.3 (1H, d, NH Tyr); 8.3 (1H, t, NH Gly); 9.3 (1H, s, OH Tyr).

### 4D. Enzymatic coupling to PhAc-Gly-Tyr-Phe-NH₂ (12)

To a solution of 3.5 g (9.5 mmol) PhAc-Gly-Tyr-OMe **(10)** and 4.65 g (28.3 mmol) L-Phe-NH₂ **(11)** in 15.6 g *t*-BuOH was added dried alcalase based on 3 mL of alcalase solution and the reaction mixture was stirred at 35 °C. After 24 h 8 g *t-*BuOH was added to increase the stirrability. After 40 h an aliquot was withdrawn from the reaction mixture and analyzed with HPLC (see below) showing that 62% of **10** had been converted to PhAc-Gly-Tyr-Phe-NH₂ **(12)** and 38% to the hydrolysis product PhAc-Gly-Tyr-OH. The reaction mixture was concentrated in vacuo and the residue partitioned between 30 g water and 45 g EtOAc. The EtOAc layer was washed with 10 g water at pH 3, dried (Na₂SO₄) and concentrated in vacuo yielding 2.5 g (5.0 mmol) of pure **12** (53% based on **10).**

¹H-NMR (DMSO-*d*₆), δ(ppm): 2.6 (1 H, dd, CH₂ Tyr or Phe); 2.7 (1 H, dd, CH₂ Tyr or Phe); 2.9 (1 H, dd, CH₂ Tyr or Phe); 3.0 (1 H, dd, CH₂ Tyr or Phe); 3.45 (2H, s, CH₂ PhAc); 3.6 (1 H, dd, CH₂ Gly); 3.8 (1 H, dd, CH₂ Gly); 4.45 (1 H, m, α-H Tyr); 4.45 (1H, m, α-H Phe); 6.6 (2H, d, CH Tyr); 7.0 (2H, d, CH Tyr); 7.1 (1H, s, NH₂); 7.2-7.3 (10H, m, PhAc, Phe); 7.4 (1H, s, NH₂); 8.1 (1H, d, NH Tyr or Phe); 8.1 (1H, d, NH Tyr or Phe); 8.3 (1 H, t, NH Gly); 9.3 (1 H, s, OH Tyr).

HPLC analysis: the coupling of **10** with **11** to form **12** was monitored with reversed-phase HPLC using the same method as described in Example 1A. Retention times for **11,** PhAc-Gly-Tyr-OH, **10** and **12** were 3.4, 7.8, 8.9 and 9.1 min, respectively.

### 4E. Enzymatic deprotection of PhAc-Gly-Tyr-Phe-NH₂ (12) to give PhAc-Gly-Tyr-Phe-OH (13)

To a suspension of 1.0 g (2.0 mmol) PhAc-Gly-Tyr-Phe-NH₂ **(12)** in 200 mL 50 mM aq. Tris/HCl buffer (pH = 7.5) containing 5 vol% DMF was added 1 mL of Peptide Amidase from *Stenotrophomonas maltophilia* (PAM; 2.87 U/mL) and the reaction mixture was stirred at 30 °C. After 24 h an aliquot was withdrawn from the reaction mixuture and analysed with HPLC (see below) showing that the conversion to PhAc-Gly-Tyr-Phe-OH **(13)** was 100%. The mixture was concentrated in vacuo and the residue partitioned between 23 g of EtOAc and 25 g of water at pH 1.0. After vigorous stirring **13** crystallized which was isolated by filtration. The water layer was extracted once more with 20 g of EtOAc and the combined EtOAc phases were dried (Na₂SO₄) and concentrated in vacuo giving a colorless oil. The oil was partitioned between 25 g CH₂Cl₂ and 25 g water at pH 1.0. The CH₂Cl₂ layer was washed with 25 g of water at pH 1.0, dried (Na₂SO₄) and concentrated in vacuo yielding another crop of **13** as a white solid. The total yield of **13** was 0.6 g (1.2 mmol, 60% based on **12).**

¹H-NMR (DMSO-*d*₆), δ (ppm): 2.5 (1H, dd, CH₂Tyr or Phe); 2.6 (1 H, dd, CH₂ Tyr or Phe); 2.9 (1 H, dd, CH₂ Tyr or Phe); 3.0 (1 H, dd, CH₂ Tyr or Phe); 3.45 (2H, s, CH₂ PhAc); 3.55 (1 H, dd, CH₂ Gly); 3.8 (1H, dd, CH₂ Gly); 4.5 (1 H, m, α-H Tyr ); 4.5 (1H, m, α-H Phe); 6.6 (2H, d, CH Tyr); 7.0 (2H, d, CH Tyr); 7.2-7.3 (10H, m, PhAc, Phe); 7.9 (1 H, d, NH Tyr or Phe); 8.2 (1H, t, NH Gly); 8.3 (1 H, d, NH Tyr or Phe); 9.2 (1 H, s, OH Tyr).

HPLC analysis: the hydrolysis of **12** to **13** was monitored with the same reversed-phase HPLC method as described in Example 1 A. Retention times for **12** and **13** were 9.1 and 9.3 min, respectively.

### 4F. Enzymatic deprotection of PhAc-Gly-Tyr-Phe-OH (13) to give H-Gly-Tyr-Phe-OH (14) and recovery of H-Gly-Tyr-Phe-OH (14).

To a solution of 50 mg (0.1 mmol) PhAc-Gly-Tyr-Phe-OH **(13)** in 10 mL aq. 0.1 M TAPS (tris(hydroxymethyl)methyl-3-aminopropane sulphonic acid) buffer of pH 9.0 was added 50 mg immobilized separase. After 30 min an aliquot was withdrawn from the reaction mixture and analysed by HPLC (see below), showing that the conversion of **13** to **14** was 100%. The enzyme was filtered off and the reaction mixture concentrated in vacuo to complete dryness. The residue contained the desired tripeptide **14** as its salt with phenylacetic acid **5.**

MS (Mass Spectrometry) confirmed the correct molecular weight of **14** (408 [M+Na]⁺; 384 [M-H]⁻).

¹H-NMR (DMSO-*d₆*), δ (ppm): 2.5 (1 H, dd, CH₂ Tyr or Phe); 2.6 (1H, dd, CH₂ Tyr or Phe); 2.9 (1H, dd, CH₂ Tyr or Phe); 3.0 (1 H, dd, CH₂ Tyr or Phe); 3.45 (2H, s, CH₂ PhAc); 3.55 (2H, s, CH₂ Gly); 4.0 (1 H, m, α-H Tyr); 4.5 (1 H, m, α-H Phe); 6.6 (2H, d, CH Tyr); 7.0 (2H, d, CH Tyr); 7.2-7.3 (5H, m, Phe).
HPLC analysis: the hydrolysis of **13** to **14** was monitored with the same reversed-phase HPLC method as described in Example 1A. Retention times for **14** and **13** were 6.6 and 9.3 min, respectively.

## Claims

1. Process for the preparation of an optionally N-protected oligopeptide alkylester comprising the step of b) reacting the corresponding optionally N-protected oligopeptide C-terminal carboxyamide with an alkyl alcohol in the presence of a peptide amidase.

2. Process according to claim 1, wherein the peptide amidase is from the flavedo of citrus fruits.

3. Process according to claim 2, wherein the peptide amidase is from the flavedo of oranges.

4. Process according to any one of claims 1-3, wherein the process is performed in a reaction medium with a water concentration between 0.5 and 50 vol%.

5. Process according to any one of claims 1-4, wherein at least 50% of the liberated NH₃ is removed from the reaction medium.

6. Process according to claim 5, wherein the NH₃ is removed by addition of a compound that complexates with NH₃.

7. Process according to claim 6, wherein the compound that complexates with NH₃ is MgHPO₄, Al₂O₃ or K₂SO₄.

8. Process according to any one of claims 1-7, wherein the alkyl alcohol is methanol.

9. Process according to any one of claims 1-8, wherein the peptide amidase is added to the reaction medium in more than one portion over time.

10. Process for the preparation of an oligopeptide comprising the process according to any one of claims 1-9.

11. Process for the preparation of an oligopeptide comprising the following steps:
b) reacting an optionally N-protected oligopeptide C-terminal carboxyamide with an alkyl alcohol in the presence of a peptide amidase according to any one of claims 1-9,
c) reacting the formed optionally N-protected oligopeptide C-terminal alkylester with an amino acid C-terminal carboxyamide or with an oligopeptide C-terminal carboxyamide in the presence of an enzyme that catalyzes peptide bond formation to form an optionally N-protected oligopeptide C-terminal carboxyamide.

12. Process according to claim 11, wherein step b) and c) are repeated until an optionally N-protected oligopeptide C-terminal carboxyamide of the desired amino acid sequence is obtained.

13. Process according to claim 11 or claim 12, wherein the optionally N-protected oligopeptide C-terminal carboxyamide used in step b), is prepared by a) reacting an optionally N-terminal protected amino acid C-terminal alkylester or an optionally N-terminal protected oligopeptide C-terminal alkylester with an amino acid C-terminal carboxyamide or with an oligopeptide C-terminal carboxyamide in the presence of an enzyme that catalyzes peptide bond formation.

14. Process according to any one of claims 11-13, further comprising the steps of
d) deprotecting the optionally N-protected oligopeptide C-terminal carboxyamide of the desired amino acid sequence on the C-terminus and/or on the N-terminus and/or - if at least one amino acid side chain protecting group is present - on at least one of the amino acid side chains and/or
e) recovering the protected or unprotected oligopeptide.

15. Process according to any one of claims 11-14, wherein the N-terminal protecting group is enzymatically introduced and/or removed.

16. Process according to claim 14, wherein the C-terminus of the optionally N-protected oligopeptide C-terminal carboxyamide is deprotected by conversion of the optionally N-protected oligopeptide C-terminal carboxyamide into the corresponding C-terminal carboxylic acid in an aqueous solution containing not more than 40 wt% alkyl alcohol in the presence of a peptide amidase.

## Patentansprüche

1. Verfahren zur Herstellung eines gegebenenfalls N-geschützten Oligopeptid-Alkylesters, wobei man im Schritt b) des Verfahrens das entsprechende C-terminale Carboxamid des gegebenenfalls N-geschützten Oligopeptids mit einem Alkylalkohol in Gegenwart einer Peptidamidase umsetzt.

2. Verfahren nach Anspruch 1, wobei die Peptidamidase aus dem Flavedo von Zitrusfrüchten stammt.

3. Verfahren nach Anspruch 2, wobei die Peptidamidase aus dem Flavedo von Orangen stammt.

4. Verfahren nach einem der Ansprüche 1-3, wobei das Verfahren in einem Reaktionsmedium mit einer Wasserkonzentration zwischen 0,5 und 50 Vol.-% ausgeführt wird.

5. Verfahren nach einem der Ansprüche 1-4, wobei mindestens 50% des freigesetzten NH₃ aus dem Reaktionsmedium entfernt werden.

6. Verfahren nach Anspruch 5, wobei das NH₃ durch Zugabe einer Verbindung, die mit NH₃ einen Komplex bildet, entfernt wird.

7. Verfahren nach Anspruch 6, wobei es sich bei der Verbindung, die mit NH₃ einen Komplex bildet, um MgHPO₄, Al₂O₃ oder K₂SO₄ handelt.

8. Verfahren nach einem der Ansprüche 1-7, wobei es sich bei dem Alkylalkohol um Methanol handelt.

9. Verfahren nach einem der Ansprüche 1-8, wobei die Peptidamidase in mehr als einer Portion mit der Zeit zu dem Reaktionsmedium gegeben wird.

10. Verfahren zur Herstellung eines Oligopeptids, umfassend das Verfahren nach einem der Ansprüche 1-9.

11. Verfahren zur Herstellung eines Oligopeptids, bei dem man die folgenden Schritte durchführt:
b) Umsetzen des C-terminalen Carboxamids eines gegebenenfalls N-geschützten Oligopeptids mit einem Alkylalkohol in Gegenwart einer Peptidamidase nach einem der Ansprüche 1-9,
c) Umsetzen des gebildeten C-terminalen Alkylesters des gegebenenfalls N-geschützten Oligopeptids mit dem C-terminalen Carboxamid einer Aminosäure oder eines Oligopeptids in Gegenwart eines Enzyms, das die Bildung einer Peptidbindung katalysiert, so dass das C-terminale Carboxamid eines gegebenenfalls N-geschützten Oligopeptids gebildet wird.

12. Verfahren nach Anspruch 11, wobei die Schritte b) und c) so lange wiederholt werden, bis das C-terminale Carboxamid eines gegebenenfalls N-geschützten Oligopeptids der gewünschten Aminosäuresequenz erhalten wird.

13. Verfahren nach Anspruch 11 oder Anspruch 12, wobei man das in Schritt b) verwendete C-terminale Carboxamid des gegebenenfalls N-geschützten Oligopeptids herstellt, indem man a) einen C-terminalen Alkylester einer gegebenenfalls N-terminal geschützten Aminosäure oder eines gegebenenfalls N-terminal geschützten Oligopeptids mit dem C-terminalen Carboxamid einer Aminosäure oder eines Oligopeptids in Gegenwart eines Enzyms, das die Bildung von Peptidbindungen katalysiert, umsetzt.

14. Verfahren nach einem der Ansprüche 11-13, bei dem man ferner in Schritt d) das C-terminale Carboxamid des gegebenenfalls N-geschützten Oligopeptids der gewünschten Aminosäuresequenz am C-Terminus und/oder am N-Terminus und/oder - falls wenigstens eine Aminosäureseitenkettenschutzgruppe vorhanden ist - an wenigstens einer der Aminosäureseitenketten entschützt und/oder e) das geschützte bzw. ungeschützte Oligopeptid gewinnt.

15. Verfahren nach einem der Ansprüche 11-14, wobei die N-terminale Schutzgruppe enzymatisch eingeführt und/oder entfernt wird.

16. Verfahren nach Anspruch 14, wobei der C-Terminus des C-terminalen Carboxamids des gegebenenfalls N-geschützten Oligopeptids durch Umwandlung des C-terminalen Carboxamids des gegebenenfalls N-geschützten Oligopeptids in die entsprechende C-terminale Carbonsäure in einer wässrigen Lösung mit nicht mehr als 40 Gew.-% Alkylalkohol in Gegenwart einer Peptidamidase entschützt wird.

## Revendications

1. Processus pour la préparation d'un ester alkylique d'oligopeptide, facultativement N-protégé, comprenant l'étape consistant à : b) faire réagir le carboxyamide C-terminal d'oligopeptide, facultativement N-protégé, avec un alcool alkylique en présence d'une peptide amidase.

2. Processus selon la revendication 1, dans lequel la peptide amidase provient du flavedo des agrumes.

3. Processus selon la revendication 2, dans lequel la peptide amidase provient du flavedo des oranges.

4. Processus selon l'une quelconque des revendications 1 à 3, dans lesquelles le processus est exécuté dans un milieu de réaction avec une teneur en eau comprise entre 0,5 et 50% vol.

5. Processus selon l'une quelconque des revendications 1 à 4, dans lequel au moins 50% du NH₃ libéré est ôté du milieu de réaction.

6. Processus selon la revendication 5, dans lequel le NH₃ est ôté par ajout d'un composé qui se complexe avec le NH₃.

7. Processus selon la revendication 6, dans lequel le composé qui se complexe avec le NH₃ est le MgHPO₄, l'Al₂O₃ ou le K₂SO₄.

8. Processus selon l'une quelconque des revendications 1 à 7, dans lequel l'alcool alkylique est le méthanol.

9. Processus selon l'une quelconque des revendications 1 à 8, dans lequel plus d'une part de peptide amidase est ajoutée au milieu de réaction dans le temps.

10. Processus pour la préparation d'un oligopeptide comprenant le processus selon l'une quelconque des revendications 1 à 9.

11. Processus pour la préparation d'un oligopeptide comprenant les étapes suivantes :
b) faire réagir un carboxyamide C-terminal d'oligopeptide, facultativement N-protégé, avec un alcool alkylique en présence d'une peptide amidase, selon l'une quelconque des revendications 1 à 9,
c) faire réagir l'ester alkylique C-terminal d'oligopeptide formé, facultativement N-protégé, avec un carboxyamide C-terminal d'acide aminé ou avec un carboxyamide C-terminal d'oligopeptide, en présence d'une enzyme qui catalyse la formation d'une liaison peptidique, pour former un carboxyamide C-terminal d'oligopeptide, facultativement N-protégé.

12. Processus selon la revendication 11, dans lequel les étapes b) et c) sont répétées jusqu'à ce que soit obtenu un carboxyamide C-terminal d'oligopeptide, facultativement N-protégé, ayant la séquence d'acides aminés souhaitée.

13. Processus selon la revendication 11 ou la revendication 12, dans lequel le carboxyamide C-terminal d'oligopeptide, facultativement N-protégé, utilisé à l'étape b) est préparé via l'étape consistant à : a) faire réagir un ester alkylique C-terminal d'acide aminé, facultativement protégé à l'extrémité N-terminale, ou bien un ester alkylique C-terminal d'oligopeptide, facultativement protégé à l'extrémité N-terminale, avec un carboxyamide C-terminal d'acide aminé ou avec un carboxyamide C-terminal d'oligopeptide en présence d'une enzyme qui catalyse la formation d'une liaison peptidique.

14. Processus, selon l'une quelconque des revendications 11 à 13, comprenant en outre les étapes consistant à : d) déprotéger le carboxyamide C-terminal d'oligopeptide, facultativement N-protégé, ayant la séquence d'acides aminés souhaitée à l'extrémité C-terminale et/ou à l'extrémité N-terminale et/ou - si au moins un groupe de protection de la chaîne latérale d'acides aminés est présent - sur au moins l'une des chaînes latérales d'acides aminés ; et/ou e) récupérer l'oligopeptide protégé ou non protégé.

15. Processus selon l'une quelconque des revendications 11 à 14, dans lequel le groupe de protection N-terminal est introduit et/ou ôté enzymatiquement.

16. Processus selon la revendication 14, dans lequel l'extrémité C-terminale du carboxyamide C-terminal d'oligopeptide, facultativement N-protégé, est déprotégée par conversion du carboxyamide C-terminal d'oligopeptide, facultativement N-protégé, en l'acide carboxylique C-terminal correspondant, dans une solution aqueuse contenant 40% en poids maximum d'alcool alkylique en présence d'une peptide amidase.
